# EUROPEAN PATENT SPECIFICATION

(11) **EP 4 467 169 B1**
(45) Date of publication and mention of the grant of the patent: **26.08.2026**
(21) Application number: 24192950.4
(22) Date of filing: 29.01.2021
(51) Int. Cl.: A61L 27/02, A61L 27/12, A61L 27/36, A61L 27/42, A61L 27/58

(54) **IMPROVED BONE GRAFT SUBSTITUTE FORMULATION**
VERBESSERTE KNOCHENTRANSPLANTATERSATZFORMULIERUNG
FORMULATION AMÉLIORÉE DE SUBSTITUT DE GREFFON OSSEUX

(30) Priority: 31.01.2020 US 202062968794 P
(43) Date of publication of application: 27.11.2024
(62) Document number(s) of the earlier application(s) in accordance with Art. 76 EPC: 21708430.0 / 4 096 730
(73) Proprietor: Wright Medical Technology, Inc., Memphis, TN 38117 (US)
(72) Inventor: MOSELEY, Jon P., Memphis, 38117 (US); BLUM, Barbara E., Memphis, 38117 (US)
(74) Representative: Carpmaels & Ransford LLP

(56) References cited:
- US-B2- 9 180 224
- HU G ET AL: "Study on injectable and degradable cement of calcium sulphate and calcium phosphate for bone repair", JOURNAL OF MATERIALS SCIENCE: MATERIALS IN MEDICINE, SPRINGER NEW YORK LLC, UNITED STATES, vol. 21, no. 2, 1 February 2010 (2010-02-01), pages 627 - 634, XP002693694, ISSN: 0957-4530, [retrieved on 20091013], DOI: 10.1007/S10856-009-3885-Z

## Description

### IMPROVED BONE GRAFT SUBSTITUTE FORMULATION

This application claims the benefit of priority to U.S. Provisional Application No. 62/968,794 filed on January 31, 2020.

### INTRODUCTION

Defects in bone structure arise in a variety of circumstances, such as trauma, disease, and surgery. There is a need for effective repair of bone defects in various surgical fields, including maxillo-craniofacial, periodontics, and orthopedics. Numerous natural and synthetic materials and compositions have been used to stimulate healing at the site of a bone defect. As with compositions used to repair other types of tissue, the biological and mechanical properties of a bone repair material are important in determining the effectiveness and suitability of the material in any particular application.

U.S. Patent No. 9,180,224 discloses bone graft cement compositions that combine a desirable absorption rate with high mechanical strength and osteoconductivity This prior art discloses a particulate composition adapted for forming a bone graft substitute cement upon mixing with an aqueous solution, including i) a calcium sulfate hemihydrate powder having a bimodal particle distribution and a median particle size of about 5 to about 20 microns, wherein the calcium sulfate hemihydrate is present at a concentration of at least about 70 weight percent based on the total weight of the particulate composition; ii) a monocalcium phosphate monohydrate powder; and iii) a beta-tricalcium phosphate powder having a median particle size of less than about 20 microns. The aqueous component that is mixed with the particulate composition of the invention is selected in order to provide the composition with a desired consistency and hardening or setting time. Typically, the aqueous solution is provided in an amount necessary to achieve a liquid to powder mass ratio (L/P) of at least about 0.2, more preferably at least about 0.21, and most preferably at least about 0.23. A preferred L/P ratio range is about 0.2 to about 0.3.. Nevertheless, the presently available synthetic bone repair formulations and kits do not present ideal functional characteristics for all bone graft applications. For many reasons, there remains a need in the art for bone graft cement formulations that combine a desirable absorption rate with ease of handling, injection, and osteoconductivity.

### SUMMARY

The present disclosure relates to a bone graft substitute formulation for treating bone cysts that have been difficult to treat with known formulations due to size and/or the location in the body. Bone cysts of a large volume (e.g., pelvic bone cysts that can have a void of about 20 mL to about 120 mL; femoral cysts that can have a void of about 20 mL to about 100 mL; and other large bone cysts up to about 200 mL) would require multiple syringes of currently available formulations made sequentially at the time of injection in order to fill the volume. Such large volume bone cysts also need a bone repair formulation that will not begin to set before the graft volume is delivered. Ideally, large volume bone cysts would be filled with 1-3 larger syringes of bone graft material in order to ensure delivery prior to setting. Moreover, a material that will not set while in a large syringe will beneficially reduce the number of injections required in the patient.

Many cysts require a second needle penetration for venting and, ideally, the cysts can be filled using narrower and longer needles or cannulas enabling minimally-sized injection sites. Conversely, small volume bone defects require a bone repair material formulation with a longer set time that can travel through a long and narrow needle or cannula to its destination without requiring excessive force for administration. Similarly, subchondral cysts, in both the foot and upper extremities, or other small bones require a second needle for venting, as well as for injection of the formulation with a narrower needle or cannula.

Accordingly, the present disclosure relates to a bone graft substitute formulation for treating bone cysts that have been difficult to treat with known formulations due to size and/or the location in the body. The disclosure also relates to kits for making the formulation, and formulations for use in treating bone injuries.
The scope of protection is defined by the claims. Any references in the description to methods of treatment refer to the formulations of the present invention for use in a method of treatment.

In a first aspect, the invention relates to a bone graft substitute formulation according to claims 1-12 comprising:
i. a particulate composition component comprising a calcium sulfate hemihydrate powder, monocalcium phosphate monohydrate powder, and a β-tricalcium phosphate powder, wherein the calcium sulfate hemihydrate powder is present in an amount from about 65 weight percent to about 85 weight percent, based on the total weight of the particulate composition component; and
ii. an aqueous component,

wherein the particulate composition component further comprises a plurality of β-tricalcium phosphate granules having a median particle size greater than the median particle size of the β-tricalcium phosphate powder;
wherein the bone graft substitute formulation has a liquid weight to powder weight ratio (L/P) of about 0.35 to about 0.40.

In a second aspect, the invention relates a kit comprising the components of the bone graft substitute formulation according to any of claims 1-12, optionally wherein the kit comprises one or more containers enclosing the particulate composition components and one or more separate containers enclosing the aqueous solution, optionally wherein the aqueous solution is sterile.

The present disclosure relates to a bone graft substitute formulation comprising a particulate composition component comprising calcium sulfate hemihydrate powder, monocalcium phosphate monohydrate powder, and β-tricalcium phosphate powder, wherein the calcium sulfate hemihydrate powder is present in an amount of about 65 weight percent to about 85 weight percent based on the total weight of the particulate composition component; and an aqueous solution component, wherein the bone graft substitute formulation has a liquid weight to powder weight ratio (L/P) of about 0.35 to about 0.40. The present disclosure also relates to a bone graft substitute formulation obtained by preparing a particulate composition component by combining calcium sulfate hemihydrate powder, monocalcium phosphate monohydrate powder, and β-tricalcium phosphate powder, wherein the calcium sulfate hemihydrate powder is present in an amount of about 65 weight percent to about 85 weight percent based on the total weight of the particulate composition component; and combining the particulate composition component with an aqueous solution component, wherein the bone graft substitute formulation has a liquid weight to powder weight ratio (L/P) of about 0.35 to about 0.40.

In at least one aspect of the present disclosure, the bone graft substitute formulation has a liquid weight to powder weight ratio (L/P) of about 0.36. In at least one aspect of the present disclosure, the bone graft substitute formulation has a liquid weight to powder weight ratio (L/P) of about 0.39.

In at least one aspect of the present disclosure, the bone graft formulation has an injection force of about 11 N to about 40 N, wherein the injection force measurement is performed using a 14 mL syringe (14.4 mm internal diameter (ID)) and an 11 gauge x 10 cm needle, and the plunger is displaced at 4.4 mm/sec. In at least one aspect of the present disclosure, the bone graft cement formulation has a Vicat set time in a dry environment of about 30 minutes to about 55 minutes. In at least one aspect of the present disclosure, the bone graft formulation has a Vicat set time in a wet environment of about 29 minutes to about 45 minutes.

In at least one aspect of the present disclosure, the aqueous solution component comprises glycolic acid in an amount of about 2.7% Wt/V to about 4.6% Wt/V, sodium hydroxide in an amount of about 1.4% Wt/V to about 2.4% Wt/V, and water. In at least one aspect of the present disclosure, the aqueous solution component comprises glycolic acid in an amount of about 2.8% Wt/V to about 3.1% Wt/V, sodium hydroxide in an amount of about 1.4% Wt/V to about 1.7% Wt/V, and water.

In at least one aspect of the present disclosure, the calcium phosphate components are present in an amount of about 14 weight percent to about 34 weight percent based on the total weight of the particulate composition component. In at least one aspect of the present disclosure, the monocalcium phosphate monohydrate powder is present in an amount of about 3 weight percent to about 7 weight percent based on the total weight of the particulate composition component. In at least one aspect of the present disclosure, the β tricalcium phosphate powder is present in an amount of about 4.1 weight percent to about 8.6 weight percent based on the total weight of the particulate composition component. The particulate composition component further comprises β-tricalcium phosphate granules. In at least one aspect of the present disclosure, the β-tricalcium phosphate granules are present in an amount of about 8 weight percent to about 12 weight percent based on the total weight of the particulate composition component. In at least one aspect of the present disclosure, the calcium sulfate hemihydrate powder is α-calcium sulfate hemihydrate powder. In at least one aspect of the present disclosure, the particulate composition component further comprises an accelerant.

In at least one aspect of the present disclosure, the bone graft substitute formulation is injectable. In at least one aspect of the present disclosure, the bone graft substitute formulation is capable of being applied as a coating for an implant. In at least one aspect of the present disclosure, the bone graft substitute formulation can be injected by hand through an 11 to 16 gauge needle up to 10 cm long in combination with a syringe up to 60 mL in volume. In at least one aspect of the present disclosure, the cement resulting from the bone graft substitute formulation has an average in vitro dissolution rate in deionized water, expressed as an average percentage of weight loss per day, of about 6% to about 15%.

The present disclosure still further relates methods of preparing the above-described formulations. The present disclosure further relates to a kit for preparing a bone graft substitute formulation comprising particulate components comprising one or more containers containing monocalcium phosphate monohydrate powder; one or more containers containing calcium sulfate hemihydrate powder and/or β-tricalcium phosphate powder; and aqueous solution components comprising one or more containers containing an aqueous solution; wherein, when the monocalcium phosphate monohydrate powder, calcium sulfate hemihydrate powder and β-tricalcium phosphate powder are combined to form a particulate composition component, the calcium sulfate hemihydrate is present in an amount of about 65 weight percent to about 85 weight percent based on the total weight of the particulate composition component; and wherein, the bone graft substitute formulation formed by the combination of the entire contents of all containers has a liquid weight to powder weight ratio (L/P) of about 0.35 to about 0.40.

In at least one aspect of the kit of the present disclosure, the aqueous solution components are in at least two containers, wherein one container contains only water. In at least one aspect of the kit of the present disclosure, the aqueous solution components are in at least two containers, wherein one container contains only glycolic acid. In at least one aspect of the kit of the present disclosure, the containers containing the monocalcium phosphate monohydrate powder and the β-tricalcium phosphate powder are two separate containers. In at least one aspect of the kit of the present disclosure, the containers containing the monocalcium phosphate monohydrate powder and the β-tricalcium phosphate powder are two separate chambers in one container. In at least one aspect of the kit of the present disclosure, the kit further comprises β-tricalcium phosphate granules in a container separate from the monocalcium phosphate monohydrate powder. In at least one aspect of the kit of the present disclosure, the calcium sulfate hemihydrate powder is α-calcium sulfate hemihydrate powder.

In at least one aspect of the kit of the present disclosure, the resulting bone graft substitute formulation has a liquid weight to powder weight ratio (L/P) of about 0.36. In at least one aspect of the kit present disclosure, the resulting bone graft substitute formulation has a liquid weight to powder weight ratio (L/P) of about 0.39. In at least one aspect of the kit present disclosure, the resulting bone graft formulation has an injection force of about 11 N to about 40 N, wherein the injection force measurement is performed using a 14 mL syringe (14.4 mm ID) and an 11 gauge x 10 cm needle, and the plunger is displaced at 4.4 mm/sec. In at least one aspect of the kit of the present disclosure, the bone graft cement formulation has a Vicat set time in a dry environment of about 30 minutes to about 55 minutes. In at least one aspect of the kit of the present disclosure, the resulting bone graft formulation has a Vicat set time in a wet environment of about 29 minutes to about 45 minutes.

In at least one aspect of the kit of the present disclosure, the aqueous solution component comprises glycolic acid in an amount of about 2.7% Wt/V to about 4.6% Wt/V, sodium hydroxide in an amount of about 1.4% Wt/V to about 2.4% Wt/V, and water. In at least one aspect of the kit of the present disclosure, the aqueous solution component comprises glycolic acid in an amount of about 2.8% Wt/V to about 3.1% Wt/V, sodium hydroxide in an amount of about 1.4% Wt/V to about 1.7% Wt/V, and water.

In at least one aspect of the kit of the present disclosure, the calcium phosphate components are present in an amount of about 14 weight percent to about 34 weight percent based on the total weight of the particulate composition component. In at least one aspect of the kit of the present disclosure, the monocalcium phosphate monohydrate powder is present in an amount of about 3 weight percent to about 7 weight percent based on the total weight of the particulate composition component. In at least one aspect of the kit of the present disclosure, the β tricalcium phosphate powder is present in an amount of about 4.1 weight percent to about 8.6 weight percent based on the total weight of the particulate composition component. In at least one aspect of the kit of the present disclosure, the β tricalcium phosphate granules are present in an amount of about 8 weight percent to about 12 weight percent based on the total weight of the particulate composition component. In at least one aspect of the kit of the present disclosure, the particulate composition component further comprises an accelerant.

In at least one aspect of the kit of the present disclosure, the resulting bone graft substitute formulation is injectable. In at least one aspect of the kit of the present disclosure, the resulting bone graft substitute formulation is capable of being applied as a coating for an implant. In at least one aspect of the kit of the present disclosure, the resulting bone graft substitute formulation can be injected by hand through an 11 to 16 gauge needle up to 10 cm long in combination with a syringe up to 60 mL in volume. In at least one aspect of the kit of the present disclosure, the cement resulting from the bone graft substitute formulation has an average in vitro dissolution rate in deionized water, expressed as an average percentage of weight loss per day, of about 6% to about 15%.

The present disclosure further relates to the bone graft substitute formulation according to any of the aspects described herein for use in a method of treating a bone defect comprising administering the bone graft substitute formulation of any of claims 1-12 to the bone defect.

In one aspect of the method for treating a bone defect disclosed herein, the bone defect is a large bone cyst having a void of up to 200 mL. In one aspect of the method for treating a bone defect disclosed herein, the bone defect is a pelvic bone cyst having a void of about 25 mL to about 120 mL. In one aspect of the method for treating a bone defect disclosed herein, the bone defect is a femoral cyst having a void of about 25 mL to about 100 mL. In one aspect of the method for treating a bone defect disclosed herein, the bone graft substitute formulation is administered with a 40 mL syringe (24.2 mm ID) and a 16 gauge x 6 cm needle. In one aspect of the method for treating a bone defect disclosed herein, the bone graft substitute formulation is administered with a 40 mL syringe (24.2 mm ID) and a 15 gauge x 4 inch needle (1 inch = 2.54 cm).
In one aspect of the method for treating a bone defect disclosed herein, the bone graft substitute formulation is administered with a 40 mL syringe (24.2 mm ID) and a 13 gauge x 10 cm needle. In one aspect of the method for treating a bone defect disclosed herein, the bone defect is a small volume bone defect such as a subchondral cyst. In one aspect of the method for treating a bone defect disclosed herein, the defect is in a small bone requiring a second needle for venting in addition to a narrow needle or cannula for injection of the formulation. In one aspect of the method for treating a bone defect disclosed herein, the bone defect has a void of about 2 mL to about 5 mL. In one aspect of the method for treating a bone defect disclosed herein, the bone graft substitute formulation is administered with a 14 mL syringe (14.4 mm ID) and a 16 gauge x 6 cm needle. In one aspect of the method for treating a bone defect disclosed herein, the bone graft substitute formulation is administered with a 14 mL syringe (14.4 mm ID) and 16 gauge x 10 cm cannula. In one aspect of the method for treating a bone defect disclosed herein, the bone graft substitute formulation is administered with a 14 mL syringe (14.4 mm ID) and a 15 gauge x 4 inch needle. In one aspect of the method for treating a bone defect disclosed herein, the bone graft substitute formulation is administered with a 14 mL syringe (14.4 mm ID) and a 13 gauge x 10 cm needle.

### BRIEF DESCRIPTION OF THE DRAWINGS

Having thus described the present disclosure in general terms, reference will now be made to the accompanying drawings, wherein:
FIG. 1 graphically illustrates the relationship between injection force of the bone graft substitute formulation and the L/P ratio of the formulation wherein the aqueous solution AS2, as described in Example 1, was used.
FIG. 2 graphically illustrates the relationship between injection force of the bone graft substitute formulation and the L/P ratio of the formulation wherein the aqueous solutions AS1, AS3₀, AS3, and AS4, as described in Example 1, were used.
FIG. 3 graphically illustrates the injection force associated with injecting about 7 cc of an exemplary graft using a 14 mL syringe with needles having different sizes, as described in Example 2.
FIG. 4 graphically illustrates the injection force associated with injecting about 25 cc of an exemplary graft using a 40 mL syringe with needles having different sizes, as described in Example 2.
FIG. 5 graphically illustrates the working time for multiple injections using a 14 mL syringe as described in Example 3.
FIG. 6 graphically illustrates the working time for multiple injections using a 40 mL syringe as described in example 3.
FIG. 7 graphically illustrates the working time for a single injection using a 14 mL syringe as described in Example 4.
FIG. 8 graphically illustrates the working time for a single injection using a 40 mL syringe as described in Example 4.

### DETAILED DESCRIPTION

Reference will now be made in detail to certain aspects of the present disclosure, examples of which are illustrated in the accompanying drawings.

Reference to "about" a value or parameter herein also includes (and describes) aspects that are directed to that value or parameter per se.

As used herein and in the appended claims, the singular forms "a," "an," and "the" include plural reference unless the context clearly indicates otherwise.

It is understood that all aspects and embodiments of the present disclosure described herein may include "comprising," "consisting," and "consisting essentially of" aspects and embodiments. It is to be understood that methods or compositions "consisting essentially of" the recited elements include only the specified steps or materials and those that do not materially affect the basic and novel characteristics of those methods and compositions.

Bone graft cement powders have been described, for example, in U.S. Patent No. 9,180,224. However, the formulations taught in U.S. Patent No. 9,180,224 are to maintain a liquid to powder weight ratio (L/P) ranging from about 0.2 to about 0.3, and preferably about 0.2 to about 0.25. Further, U.S. Patent No. 9,180,224 discusses using an accelerant in the powder if adjusting the set time to set faster is desired. It has been discovered, surprisingly, that a bone graft substitute formulation with a liquid to powder ratio (L/P) of about 0.35 to about 0.40, such as about 0.36 to about 0.39, has a significantly longer set time. Further surprisingly, despite the presence of additional liquid in the disclosed formulation with an L/P of about 0.35 to about 0.40, the corresponding hardened cements have a comparable in vitro dissolution rate as the hardened cements formed with formulations in the art with lower L/P ratios, measured as set forth in the Examples below. Moreover, the formulations with an L/P ratio of about 0.35 to about 0.40 require significantly lower injection forces required for administration.

The improved workability characteristics (longer set time and lower injection forces) of the formulation described herein allow for use in larger syringes for filling large bone cyst volume, as well as allow for use in long, narrow needles for small bone defects and for larger defects for which very minimal disruption of tissue surrounding the bone is desired or required. Moreover, the formulation disclosed herein with its longer set time and lower injection forces can treat both large volume bone cysts as well as fragile bone injuries, such as subchondral cysts, without compromising its dissolution rate, and eventual replacement with new bone. The comparable dissolution rates allow the disclosed formulation to provide the same absorption behavior of the prior art formulations once set inside the bone needing repair. As discussed in more detail below, it is believed that the bone graft substitute formulation of the present disclosure will become a highly porous matrix of calcium phosphate material after being administered in vivo due to the relatively quick absorption of the calcium sulfate component of the mixture. The remaining porous matrix of calcium phosphate provides excellent scaffolding for bone ingrowth during the natural healing process.

The formulation described herein is made with a particulate composition useful as a bone graft substitute that begins to harden or set after mixing with an aqueous solution. As used herein, the terms "set time," "Vicat set time," "Vicat needle drop test," and the like, refer to measurements according to the "Vicat Needle Drop Test for Setting Time Measurement" described in detail in the "Experimental" section below. The particulate composition includes a calcium sulfate hemihydrate (hereinafter "CSH") powder and a brushite-forming calcium phosphate mixture comprising monocalcium phosphate monohydrate (hereinafter "MCPM") powder and a β-tricalcium phosphate (hereinafter "β-TCP") powder. Inclusion of β-TCP granules provides a graft with triphasic absorption.

Use of the formulation disclosed herein produces a bone graft substitute cement comprising calcium sulfate dihydrate (hereinafter "CSD"), which is the product of the reaction between CSH and water. The CSD component of the cement confers good mechanical strength to the cement, stimulates bone growth, and is relatively quickly absorbed, such that an interconnected porous structure of the cement is quickly created upon implantation. Thus, the CSD component of the cement can be rapidly replaced with bone tissue ingrowth into the implant site.

The two calcium phosphate components react to form brushite upon mixing with an aqueous solution. The presence of the brushite in the cement slows the absorption rate of the bone graft substitute cement as compared to a cement comprising CSD only. Thus, the biphasic bone graft substitute formulation of the present disclosure provides a dual absorption rate defined by the CSD component and the brushite component.

The particulate composition of the formulation disclosed herein comprises a CSH powder in an amount of from about 65 weight percent based on the total weight of the particulate composition component to about 85 weight percent. For example, the CSH powder is present in an amount of from about 70 weight percent to about 80 weight percent, such as about 75 weight percent.
In at least one aspect of the present disclosure, the CSH for the formulation disclosed herein is α-calcium sulfate hemihydrate, which exhibits higher mechanical strength as compared to the beta form upon setting to form CSD. The CSH portion of the particulate composition is important for providing mechanical strength to the resulting bone graft substitute cement, as well as contributing to the ability to set or harden in a relatively short period of time. As is known in the art, CSH has the formula CaSO₄•½H₂O, and will react with water to form calcium sulfate dihydrate (CaSO₄•H₂O). It is believed that the presence of CSD in the bone graft substitute cement formed with the formulation of the present disclosure contributes to rapid regeneration of bone tissue at the site of the bone defect.

In one aspect, the CSH powder further includes an accelerant capable of accelerating the conversion of CSH to the dihydrate form. Examples of accelerants include calcium sulfate dihydrate, potassium sulfate, sodium sulfate, or other ionic salts. In one aspect of the present 4845-0307-7594.1 disclosure, the accelerant is calcium sulfate dihydrate crystals (available from U.S. Gypsum) coated with sucrose (available from VWR Scientific Products). The accelerant is typically present in an amount of up to about 1.0 weight percent, based on the total weight of the particulate composition component. In some aspects, the particulate composition includes from about 0.001 to about 0.5 weight percent of the accelerant, more typically between about 0.01 and about 0.3 weight percent.

The calcium phosphate portion of the particulate composition component of the present disclosure comprises a MCPM powder (Ca(H₂PO₄)₂•H₂O) and a β-TCP powder (Ca₃(PO₄)₂). As understood in the art, the main reaction product of MCPM and β-TCP is brushite, otherwise known as dicalcium phosphate dihydrate (CaHPO₄•H₂O) (DCPD). The brushite-forming powders may also participate in other reactions that would result in the formation of certain calcium phosphates with a greater thermodynamic stability than DCPD, such as hydroxyapatite, octacalcium phosphate, and the like. A certain amount of the β-TCP powder may also remain unreacted in the cement.

As would be understood, MCPM is the hydrate form of monocalcium phosphate (MCP). As used herein, reference to MCPM is intended to encompass MCP, which is simply the anhydrous form of MCPM that releases the same number of calcium and phosphoric acid ions in solution. However, if MCP is used in place of MCPM, the amount of water used to form the bone graft substitute cement would need to be increased to account for the water molecule missing from MCP (if it is desired to produce precisely the same dissolution product as formed when using MCPM).

As noted herein, the brushite component of the bone graft substitute cement of the present disclosure serves to slow the in vivo absorption of the bone graft substitute cement as compared to a calcium sulfate cement. In turn, the slowing of the absorption rate with the brushite component may enable the bone graft substitute cement to provide structural support at the site of the bone defect for longer periods of time, which can aid the healing process in certain applications. Although not bound by any particular theory of operation, it is believed that the bone graft substitute formulation of the present disclosure will become a highly porous matrix of calcium phosphate material after being administered in vivo due to the relatively quick absorption of the calcium sulfate component of the mixture. The remaining porous matrix of calcium phosphate provides excellent scaffolding for bone ingrowth during the natural healing process.

The amount of MCPM powder and β-TCP powder present in the particulate composition component can vary and depends primarily on the amount of brushite desired in the bone graft substitute cement. The brushite-forming calcium phosphate composition (i.e., the combined amount of MCPM and β-TCP powders) will typically be present in an amount of about 8 to about 34 weight percent based on the total weight of the particulate composition component, for example from about 10 to about 20 weight percent,-such as about 15 weight percent. The relative amounts of MCPM and β-TCP can be selected based on their equimolar, stoichiometric relationship in the brushite-forming reaction. In one aspect, the MCPM powder is present in an amount of about 3 to about 7 weight percent, based on the total weight of the particulate composition component, and the β-TCP is present in an amount of about 4.1 to about 8.6 weight percent.

Storage of the brushite-forming calcium phosphate powders together in a homogenous mixture can result in reduction in the amount of brushite produced upon mixing the particulate composition component with the aqueous solution component to form the bone graft substitute cement, which in turn, can alter the properties of the bone graft substitute formulation in an undesirable manner. As a result, in at least one aspect, the two calcium phosphate powders are either packaged together in a dry environment and hermetically sealed against moisture invasion during storage or are packaged separately during storage. In at least one aspect, the two calcium phosphate powders are packaged separately, wherein each powder is either packaged alone with no other components of the particulate composition of the present disclosure or in admixture with one or more of the remaining components (e.g., the CSH powder).

The particulate composition component includes a plurality of β-TCP granules having a median particle size greater than the median particle size of the β-TCP powder. The β-TCP granules typically have a median particle size of about 75 to about 1,000 microns, such as about 100 to about 400 microns, and for instance about 180 to about 240 microns. The granules serve to further reduce the absorption rate of the bone graft substitute cement and contribute to scaffold formation. The β-TCP granules are typically present in an amount of up to about 20 weight percent, based on the total weight of the particulate composition component, such as up to about 15 weight percent based on the total weight of the particulate composition component, for example up to about 10 weight percent. In at least one aspect, the β-TCP granules are present in an amount of about 8 to about 12 weight percent of the particulate composition component. The β-TCP granules can provide a relatively inert third phase in the final cement that exhibits an even slower absorption rate than the brushite formed by reaction of the MCPM and the β-TCP powder. Thus, the presence of the granules can further alter the absorption profile of the resulting bone graft substitute cement.

Both the β-TCP granules and the β-TCP powder used in the present disclosure can be formed using a commercially available β-TCP powder as a starting material, such as β-TCP powder available from Plasma Biotal Ltd. (Derbyshire, UK). In one aspect, the β-TCP components of the particulate composition are formed by first wet milling a commercially available β-TCP powder in a ball mill to a median particle size of less than 1.0 micron and then draining the resulting slurry through a strainer to remove the milling media. Thereafter, the solid cake of β-TCP can be separated from any remaining liquid components using any of a variety of techniques known in the art, such as centrifuging, gravity separation, filter pressing, evaporation, and the like. The dry cake is then processed through a series of sieves in order to produce two separate β-TCP components having different median particle sizes. The dried cake of β-TCP is typically milled either during or prior to sieving in order to fragment the cake. In at least one aspect, the system of sieves produces a β-TCP component having a particle size range of about 125 to about 355 microns in a green (i.e., unfired) state and another β-TCP component having a particle size range of about 75 to about 355 microns in a green state. Thereafter, the two β-TCP components are sintered, and thereby densified, by heat treatment in a furnace. In one aspect, the furnace treatment involves heating the β-TCP powder components on an alumina plate at a temperature of about 1100-1200°C. for about three hours. It is typical to ramp the temperature up to the desired sintering temperature and ramp the temperature back down during the cooling period at a rate no greater than about 5-6°C. per minute.

Following the sintering process, the densified β-TCP granules having had a green state particle size of about 125 to about 355 microns can be used as the granule component of the particulate composition. The sintered β-TCP component having had a green (i.e., unfired) state particle size of about 75 to about 355 microns can be dry milled in a ball mill for approximately one to four hours in order to form the β-TCP powder having a median particle size of less than about 20 microns, which can then be used in the particulate composition component as described herein.

The aqueous component that is mixed with the particulate composition component to form the bone graft substitute formulation of the present disclosure is selected in order to provide the formulation with a desired consistency and hardening or setting time. The aqueous solution is provided in an amount necessary to achieve a liquid to powder mass ratio (L/P) of at least about 0.35, more preferably at least about 0.36. A preferred L/P range is about 0.35 to about 0.40, more preferably about 0.36 to about 0.39.

Examples of suitable aqueous components include water (e.g., sterile water, deionized water) and solutions thereof, with glycolic acid, optionally including one or more additives selected from the group consisting of sodium chloride, potassium chloride, sodium sulfate, potassium sulfate, EDTA, ammonium sulfate, ammonium acetate, and sodium acetate. In one aspect, the aqueous mixing solution used is a saline solution or a phosphate buffered saline solution. An exemplary aqueous solution is water, such as Water for Irrigation available from Baxter International (Deerfield, Ill.) and others. Another aqueous solution is 0.9% NaCl saline solution available from Baxter International (Deerfield, Ill.) and others. In at least one aspect, the mixing solution is a 0.6 M solution of glycolic acid neutralized to a pH of 7.0 using NaOH. In at least one aspect, the aqueous solution is about 0.36 M solution of glycolic acid neutralized to a pH of 7.0 using NaOH. In at least one aspect, the aqueous solution is about 0.39 M solution of glycolic acid neutralized to a pH of 7.0 using NaOH. In at least one aspect, the aqueous solution is about 0.4 M solution of glycolic acid neutralized to a pH of 7.0 using NaOH. In at least one aspect, the aqueous solution is about 0.5 M solution of glycolic acid neutralized to a pH of 7.0 using NaOH. In at least one aspect, the aqueous solution comprises a combination of glycolic acid in an amount of about 2.7% weight/volume (Wt/V) to about 4.6% Wt/V, sodium hydroxide in an amount of about 1.4% Wt/V to about 2.4% Wt/V, and water. In at least one aspect, the aqueous solution comprises a combination of glycolic acid in an amount of about 2.8% Wt/V to about 3.1% Wt/V, sodium hydroxide in an amount of about 1.4% Wt/V to about 1.7% Wt/V, and water. In at least one aspect, the water is deionized water. Reference to glycolic acid herein encompasses both the free acid and salt forms.

A bone graft substitute formulation according to the present disclosure can be formed by mixing the particulate composition component with the aqueous solution component using manual or mechanical mixing techniques and apparatus known in the art. In one aspect of the disclosure, the components of the formulation are mixed at atmospheric pressure and at a temperature that will not result in freezing of the aqueous component of the mixture or significant evaporation In another aspect of the disclosure, the components of the formulation are mixed below atmospheric pressure (e.g., under vacuum). Following mixing, the homogenous composition typically has a paste-like consistency, although the viscosity and flowability of the mixture can vary depending on the additives therein. The bone graft substitute formulation can be mixed in a delivery device, or can be mixed in a clean vessel and then transferred to a delivery device, such as a syringe, and injected into a target site, for example, to fill in cracks or voids of a bone defect. In some aspects, the formulation can be injected by hand compression through an 11 to 16-gauge needle up to, for example, 10 cm long in combination with syringes up to 60 mL in volume. Non-limiting examples of needle sizes to be used with 40 mL syringes include 16 gauge x 6 cm needle; 16 gauge x 10cm cannula; 15 gauge x 4 inch needle; 13 gauge x 10 cm needle; and larger needles with a similar length.

As discussed herein, the bone graft substitute formulations of the present disclosure exhibit longer setting times according to the Vicat needle drop test than art formulations with lower liquid to powder ratios, but maintain comparable dissolution rates. As used herein, the terms "set time," "Vicat needle drop test," "Vicat set time," and the like, refer to measurements according to the "Vicat Needle Drop Test for Setting Time Measurement" described in detail in the "Experimental" section below. The bone graft substitute formulations of the present disclosure will generally exhibit a Vicat set time in a dry environment of about 30 to about 55 minutes, more preferably about 37 to about 45 minutes. The bone graft substitute formulations of the present disclosure will generally exhibit a Vicat needle set time in a wet environment of about 29 to about 45 minutes, more preferably about 30 to about 37 minutes. Setting of the formulation can occur in a variety of environments, including air, water, in vivo, and under any number of in vitro conditions.

As discussed herein, the bone graft substitute formulations of the present disclosure maintain comparable cement dissolution rates as the art formulations with lower liquid to powder ratios. The dissolution rate of the cement's lattice structure can lead to timely subsequent new bone growth within the defect. As used herein, the terms "dissolution rate," "cement dissolution rate," "in vitro dissolution rate," and the like, refer to the measurements according to the "Cement Dissolution Test Measurement" described in detail in the "Experimental" section below. The bone graft substitute cement from the formulation as disclosed herein exhibits an average in vitro dissolution rate in deionized water, expressed as an average percentage of weight loss per day, of about 6% to about 15%, measured as set forth below. The in vitro dissolution rate in deionized water, expressed as an average percentage of weight loss per day, is calculated by subtracting the percent remaining after four days (as determined by the cement dissolution test) from 100 and dividing by four.

The bone graft substitute formulation of the present disclosure has an injection force of about 11 N to about 40 N, wherein the injection force measurement is performed using a 14 mL syringe (14.4 mm inner diameter (ID)) and an 11 gauge x 6 cm needle, and the plunger is displaced at 4.4 mm/sec. As used herein, "injection force" refers to the measurements according to the "Injection Force" described in detail in the "Experimental" section below.

The bone graft substitute formulations disclosed herein may advantageously have a prolonged working time when used in the present methods. The longer working time is beneficial during a surgical procedure since it provides additional time and flexibility to the surgeon and reduces waste of bone substituting agents and surgical delays due to preparing additional bone graft substitute formulations. The "working time" as used herein is described in the examples, and may depend on the volume the syringe (e.g., 14 mL or 40 mL) as well as the volume of bone graft substitute formulation used.

The bone graft substitute formulation of the present disclosure can further include other additives known in the art. The additives can be added as a solid or liquid to either the particulate composition of the present disclosure or the aqueous mixing solution. One example of an additive for the calcium sulfate composition is a plasticizer designed to alter the consistency and setting time of the composition. Such a plasticizing ingredient can retard the setting of calcium sulfate hemihydrate pastes, thereby increasing the time it takes for the composition to set following mixing with an aqueous solution. Exemplary plasticizers include glycerol and other polyols, vinyl alcohol, stearic acid, hyaluronic acid, cellulose derivatives and mixtures thereof. Alkyl celluloses are particularly preferred as the plasticizer ingredient. Exemplary alkyl celluloses include methylhydroxypropylcellulose, methylcellulose, ethylcellulose, hydroxyethylcellulose, hydroxypropylcellulose, hydroxypropylmethylcellulose, carboxymethylcellulose, cellulose acetate butyrate, and mixtures or salts thereof.

Exemplary additives also include biologically active agents. As used herein, the term "biologically active agent" is directed to any agent, drug, compound, composition of matter or mixture that provides some pharmacologic affect that can be demonstrated in vivo or in vitro. Examples of biologically active agents include, but are not limited to, peptides, proteins, enzymes, small molecule drugs, dyes, lipids, nucleosides, oligonucleotides, polynucleotides, nucleic acids, cells, viruses, liposomes, microparticles, and micelles. It includes agents that produce a localized or systemic effect in a patient.

In at least one aspect, classes of biologically active agents include osteoinductive or osteoconductive materials, antibiotics, chemotherapeutic agents, pesticides (e.g., antifungal agents and antiparasitic agents), antivirals, anti-inflammatory agents, and analgesics. Exemplary antibiotics include ciprofloxacin, tetracycline, oxytetracycline, chlorotetracycline, cephalosporins, aminoglycosides (e.g., tobramycin, kanamycin, neomycin, erythromycin, Vancomycin, gentamycin, and streptomycin), bacitracin, rifampicin, N-dimethylrifampicin, chloromycetin, and derivatives thereof. Exemplary chemotherapeutic agents include cis-platinum, 5-fluorouracil (5-FU), taxol and/or taxotere, Ifosfamide, methotrexate, and doxorubicin hydrochloride. Exemplary analgesics include lidocaine hydrochloride, bipivacaine and non-steroidal anti-inflammatory drugs such as ketorolac tromethamine. Exemplary antivirals include gancyclovir, Zidovudine, amantidine, Vidarabine, ribaravin, trifluridine, acyclovir, dideoxyuridine, antibodies to viral components or gene products, cytokines, and interleukins. An exemplary antiparasitic agent is pentamidine. Exemplary anti-inflammatory agents include C-1-anti-trypsin and C-1-antichymotrypsin.

Useful antifungal agents include diflucan, ketaconizole, nystatin, griseofulvin, mycostatin, miconazole and its derivatives as described in U.S. Pat. No. 3,717,655; bisdiguanides such as chlorhexidine; and more particularly quaternary ammonium compounds such as domiphen bromide, domiphen chloride, domiphen fluoride, benzalkonium chloride, cetyl pyridinium chloride, dequalinium chloride, the cis isomer of 1-(3-chloroallyl)-3,5,7-triaza-1-azoniaadamantane chloride (available commercially from the Dow Chemical Company under the trademark Dowicil 200) and its analogues as described in U.S. Pat. No. 3,228,828, cetyl trimethyl ammonium bromide as well as benzethonium chloride and methylbenzethonium chloride such as described in U.S. Pat. Nos. 2,170,111; 2,115,250; and 2,229,024; the carbanilides and salicylanilides such 3,4,4-trichlorocarbanilide, and 3,4,5-tribromosalicylanilide; the hydroxydiphenyls such as dichlorophene, tetrachlorophene, hexachlorophene, and 2,4,4-trichloro-2'-hydroxydiphenylether, and organometallic and halogen antiseptics such as zinc pyrithione, silver sulfadiazone, silver uracil, iodine, and such as described in U.S. Pat. Nos. 2,710,277 and 2,977,315, and polyvinylpyrrolidone such as described in U.S. Pat. Nos. 2,706,701, 2,826,532 and 2,900,305.

As used herein, the term "growth factors" encompasses any cellular product that modulates the growth or differentiation of other cells, particularly connective tissue progenitor cells.
The growth factors that may be used in accordance with the present disclosure include, but are not limited to, fibroblast growth factors (e.g., FGF-1, FGF-2. FGF-4); platelet-derived growth factor (PDGF) including PDGF-AB, PDGF-BB and PDGF-AA; bone morphogenic proteins (BMPs) such as any of BMP-1 to BMP-18; osteogenic proteins (e.g., OP-1, OP-2, or OP-3); transforming growth factor-O, transforming growth factor-B (e.g., (B1, B2, or B3); LIM mineralization proteins (LMPs); osteoid-inducing factor (OIF); angiogenin(s); endothelins; growth differentiation factors (GDF's); ADMP 1; hepatocyte growth factor and keratinocyte growth factor, osteogenin (bone morphogenetic protein-3); heparin-binding growth factors (HBGFs) such as HBGF-1 and HBGF-2; the hedgehog family of proteins including indian, sonic, and desert hedgehog; interleukins (IL) including IL-1 thru -6; colony-stimulating factors (CSF) including CSF-1, G-CSF, and GM-CSF; epithelial growth factors (EGFs); and insulin-like growth factors (e.g., IGF-I and -II); demineralized bone matrix (DBM); cytokines; osteopontin; and osteonectin, including any isoforms of the above proteins. Particulate DBM is a preferred osteoinductive additive.

The biologically active agent may also be an antibody. Suitable antibodies, include by way of example, STRO-1, SH-2, SH-3, SH-4, SB-10, SB-20, and antibodies to alkaline phosphatase. Such antibodies are described in Haynesworth et al., Bone (1992), 13:69-80; Bruder, S. et al., Trans Ortho Res Soc (1996), 21:574; Haynesworth, S. E., et al., Bone (1992), 13:69-80; Stewart, K., et al, J Bone Miner Res (1996), 11 (Suppl.):S142; Flemming JE, et al., in "Embryonic Human Skin." Developmental Dynamics. 212:119-132, (1998); and Bruder S. P. et al., Bone (1997), 21(3): 225-235.

Other examples of biologically active agents include bone marrow aspirate, platelet concentrate, blood, allograft bone, cancellous bone chips, synthetically derived or naturally derived chips of minerals such as calcium phosphate or calcium carbonate, mesenchymal stem cells, and chunks, shards, and/or pellets of calcium sulfate.

The present disclosure also relates to bone graft substitute kits comprising the components of the formulation of the present disclosure. Typically, the kit comprises one or more containers enclosing the particulate composition components as described herein and one or more separate containers enclosing a sterile aqueous solution. The kit will typically contain a written instruction set describing a method of using the kit. In at least one aspect of the present disclosure, the bone-graft substitute kit will comprise an apparatus for mixing the particulate composition components with the aqueous solution in order to form the bone graft formulation, such as a vacuum mixing apparatus. Additionally, the kit will typically include a device for delivering the bone graft formulation to the site of the bone defect, such as an injection device (e.g., a needle and syringe).

In at least one aspect, the particulate composition components and the sterile aqueous solution components will be sterilized by irradiation prior to packaging in the kit.

As noted herein, in certain aspects, the kit of the present disclosure will separate the two calcium phosphate powder components into different containers or compartments to avoid reaction during storage. There are a number of packaging configurations that can accomplish this goal. For example, in one aspect, the kit includes one container for CSH powder, one container for β-TCP powder, and one container for MCPM powder. In another aspect, the kit includes two containers for the particulate composition components, one including β-TCP powder and the CSH component and a second containing MCPM powder. In yet another aspect, the MCPM powder is packaged in a separate container by itself, and the β-TCP powder and the CSH powder are packaged together, but in separate compartments. In at least one aspect, CSH is α-calcium sulfate hemihydrate powder. In at least one aspect, the kit further comprises β-tricalcium phosphate granules.

In any of the aspects described herein, any of the particulate containers can further include the crystalline powder of glycolic acid salt and/or β-TCP granules, or those components could be packaged separately in their own containers. When present, the accelerant adapted to accelerate conversion of CSH to CSD is typically in admixture with the CSH powder. In at least one aspect, the kit comprises one container enclosing the MCPM powder, and a second container enclosing the remaining particulate ingredients in admixture. Such as one or more of the CSH powder, the CSH accelerator, the β-TCP powder, the β-TCP granules, and the glycolic acid crystalline powder.

In at least one aspect, the powdered form of glycolic acid is packaged separately so that it can be reconstituted in the aqueous solution, if desired, prior to mixing the solution with the remaining particulate components. However, the aqueous solution component of the kit may also contain glycolic acid in solution form. In at least one aspect, the glycolic acid is added after radiation sterilization of the aqueous component of the kit.

It will be important to utilize all of the aqueous solution component packaged in the kit in order to ensure that consistent setting times are achieved. In at least one aspect, the aqueous solution component is packaged in a highly hydrophobic container, such as a glass syringe or other glass container, that is less prone to retention of residual solution in amounts that will cause changes in the performance characteristics of the bone graft substitute cement.

The present disclosure also provides methods for treating a bone defect involving administering a bone graft substitute formulation as described herein to the site of the bone defect. The bone graft substitute formulation is administered in flowable form following mixing of the particulate composition component with the aqueous solution component, such as through an injection device, prior to setting. In at least one aspect, the bone defect is a large volume bone cyst, for example pelvic bone cysts having a void of about 25 mL to about 120 mL; femoral cysts having a void of about 25 mL to 100 mL; and other large bone cysts up to about 200 mL. In at least one aspect the administration can occur with hand compression of a 40 mL syringe (24.2 mm ID) and through a needle such as a 16 gauge, 6 cm needle; a 15 gauge x 4 inch needle; a 13 gauge x 10 cm needle; or larger needles with a similar length. In at least one aspect, the bone defect is a small volume bone defect such as a subchondral cyst, for instance in the foot or upper extremities. In at least one aspect, the defect is in a small bone requiring a second needle for venting in addition to a narrow needle or cannula for injection of the formulation. In at least one aspect the bone defect is about 2 mL to about 5 mL in size, and administration can occur with hand compression of a 14 mL syringe (14.4 mm ID) and through a needle such as a 18 gauge x 6 inch needle; a 16 gauge x 6 cm needle; a 16 gauge x 10 cm cannula; a 15 gauge x 4 inch needle; a 13 gauge x 10 cm needle; or larger needles with a longer length.

In another aspect, the bone graft substitute formulation of the present disclosure can be incorporated onto or into an orthopedic implant (for example as a coating), such as any of the various devices adapted for joint replacement. The bone graft substitute formulation is typically incorporated into such devices as an outer coating or as a filling material within the pores of a porous outer component of the device. In this aspect, the bone graft substitute formulation facilitates bone in-growth in the area surrounding the implanted device. Exemplary orthopedic implants include knee replacement devices (e.g., constrained or non-constrained knee implant devices, hinged knee devices, metallic plateau knee devices, and patellar devices), hip replacement devices (e.g., acetabular components and femoral components), elbow replacement devices (e.g., constrained, semi-constrained, and non-constrained devices), upper femoral devices, upper humeral devices, wrist replacement devices (e.g., semi-constrained 2- and 3-part articulation devices), shoulder devices, passive tendon devices, spinal devices (e.g., thoracolumbar spinal fixation devices, cervical spinal fixation devices, and spinal fusion cages), finger/toe devices, diaphysis devices, and K-wires.

The present disclosure will be further illustrated by the following non-limiting examples.

### EXPERIMENTAL

The following examples demonstrate the improved workability (longer set time and lower injection force) of the bone substitute graft formulation while maintaining the desired dissolution properties of the hardened bone graft material. The improved workability characteristics allow for use in larger syringes for filling large bone cyst volume, as well as allow for use in long, narrow needles for small bone defects and/or for larger defects for which very minimal disruption of tissue surrounding the bone is desired or required.

### Vicat Needle Drop Test for Setting Time Measurement - Dry Environment

Setting times were measured using a Vicat needle that was 1 mm in diameter, 5 cm long, and which possessed a total weight of 300 g. A more detailed description of the Vicat apparatus can be found in ASTM C-472. The sample being tested was mixed in a manner that a homogeneous, flowable paste was created. The sample sizes for the Vicat needle drop test ranged about 3 cc to about 5 cc of material tapped down to a cake in an approximately 5 mL polyethylene cup. Alternatively, the sample sizes for the Vicat needle drop test ranged about 10 cc to about 15 cc of material tapped down to a cake in an approximately 20 mL polyethylene cup. Set time according to the Vicat needle drop test is defined as the amount of time elapsed between the time the aqueous solution contacted the particulate composition and the time the Vicat needle would not pass through 50% of the height of a cement sample upon being dropped from the upper surface of the sample. The needle was allowed to fall under its own weight, under gravity alone, through a line perpendicular to the top and bottom, flat faces of the cylinder-shaped sample cake. The needle was typically dropped every 60 seconds after the first drop and every 30 seconds when the set-time was close. Cups, mixing equipment, and material transfer equipment were not reused.

### Vicat Needle Drop Test for Setting Time Measurement - Wet Environment

Setting times were measured using a Vicat needle that is 1 mm in diameter, 5 cm long, and which possessed a total weight of 300 g. A more detailed description of the Vicat apparatus can be found in ASTM C-472. The sample being tested was mixed in a manner that a homogeneous, flowable paste was created. The sample sizes for the Vicat needle drop test ranged from about 3 cc to about 5 cc of material tapped down to a cake in an approximately 5 mL polyethylene cup. Alternatively, the sample sizes for the Vicat needle drop test ranged about 10 cc to about 15 cc of material tapped down to a cake in an approximately 20 mL polyethylene cup. For wet specimen testing, a narrow channel was created between the sample and the 20 mL polyethylene cup by scraping a small spatula at the inside surface of the polyethylene cup. Using a small syringe, the gap was filled with bovine calf serum warmed to 37°C; a layer of serum up to approximately 1mm thick flowed onto the top of the specimen. Set time according to the Vicat needle drop test is defined as the amount of time elapsed between the time the aqueous solution contacted the particulate composition and the time the Vicat needle would not pass through 50% of the height of a cement sample upon being dropped from the upper surface of the sample. The needle was allowed to fall under its own weight, under gravity alone, through a line perpendicular to the top and bottom, flat faces of the cylinder-shaped sample cake. The needle was typically dropped every 60 seconds after the first drop and every 30 seconds when the set-time was close. Cups, mixing equipment, and material transfer equipment were not reused.

### Cement Dissolution Test Measurement

Dissolution rate of the material was determined through the following methodology. Specimens were cast in silicone molds to a size of 4.8 mm outer diameter and 3.3 mm tall cylinders. A 3.3 mm thick sheet of silicone containing cylindrical voids was used as a mold. The cylindrical voids were 4.8 mm in outer diameter and 3.3 mm tall, and oriented such that the circular faces of the void were parallel and in the same plane as the surfaces of the silicone sheet.

A thin sheet of polyethylene was laid on a table. A polyethylene mesh was placed on top of the polyethylene sheet; sheet and mesh were of same dimensions (excluding thickness) and positioned such that the mesh masked the sheet from the top. Next a silicone mold of smaller dimensions was placed on top of the mesh (excluding thickness). No part of the mold hung off the edge of the mesh or sheet.

The material to be tested was then mixed together to form a homogeneous paste. The paste was then wiped across the top of the mold using a spatula in a manner that the voids were packed with the material. The mesh allowed air to be displaced out of the void as the mold was filled. Several wipes were performed to assure that the material completely penetrated to bottom of the mold and extruded out through the mesh and onto the lower polyethylene sheet. A final wipe with the spatula across the top of the mold was performed to remove the majority of excess material and produce smooth top faces for the specimens.

Another polyethylene sheet of the same dimensions as the first was then placed across the top of the mold, such that it completely covered the top of the mold. This sheet was then gently pressed against the mold using a finger in a gentle rubbing motion.

The entire system: sheet, mesh, mold, and sheet, was then picked up as a whole and flipped over in a manner such that the original top was now facing down. The top polyethylene sheet, originally the bottom, and mesh were removed and the spatula was again used to wipe material into voids in the tops (previously bottoms) of the specimens created from air removal. A final wipe with the spatula across the top of the mold was performed to remove the majority of excess material. The sheet (no mesh) was returned to the top of the mold.

The specimens were left in the mold to cure for a minimum of 8 hours after the second polyethylene sheet had been placed in direct contact with the specimens and mold (no mesh). After at least 8 hours had passed, the specimens were demolded by hand. Any flash remaining attached to pellet faces were removed by rolling the specimen between fingers. All defective specimens were disqualified from the test and discarded. A defective specimen was defined as a specimen not exhibiting a cylindrical shape, which could be caused by entrapped air, defects created upon demolding, and/or physical damage to the specimen itself.

All specimens which were not defective were spread across a pan or tray in a monolayer. The pan or tray and specimens were then dried in an oven at 40° C for a minimum of 4 hours, and then removed from the oven and allowed to cool for 30 minutes in room conditions (21-27°C.; 20-50% relative humidity). From the specimens created, five (5) specimens were arbitrarily chosen to be used for dissolution testing. Each specimen chosen was paired with a clean cylindrical fritted glass extraction thimble of the following dimensions: approximately 85-90 mm overall height, 4 mm fitted glass base (40-60 micron pores) located 80 mm from top of thimble, 25 mm outer diameter, and 22 mm inner diameter. The mass of each extraction thimble was measured (0.01 mg) and noted. The mass of each specimen was measured (0.01 mg) and noted. A polyethylene bottle (300 mL) was designated to each pair (specimen and thimble). The bottle had dimensions that allowed thimble and specimen to easily be placed in and removed from bottle and upon filling with 275 mL of water, which created a column of water that was taller than the thimble. The bottle was filled with 275 mL of deionized water at room temperature (21-27°C).

The specimen was placed into its corresponding thimble and the thimble was lowered into the bottle; care was taken to keep any part of the material from escaping from the thimble. The bottle was capped and placed into a water bath at 37°C with no agitation and the time is noted. 24 hours after the specimen had been in the water, the thimble containing the specimen was retrieved. The water was allowed to drain out of the thimble through the fritted glass base. The thimble containing the specimen was then dried for 4 hours in a 40° C oven or until completely dried (determined gravimetrically). The thimble containing the specimen was then allowed to cool down for 30 minutes at room conditions (21-27 C.; 20-50% relative humidity).

The thimble-containing the pellet was then weighed to an accuracy of 0.01 mg. Subtracting the known empty thimble mass from the mass of the combination resulted in the mass of the specimen alone. Subtracting this mass from the initial specimen mass produced the mass lost to dissolution. This mass lost divided by the specimen initial mass, and the product of that multiplied by 100 resulted in the % mass lost from dissolution. At this point the thimble containing the pellet was returned to the bottle containing fresh deionized water (275 mL) at room temperature (21-27°C.), and the bottle was capped and returned to the water bath. After 24 hours the drying and weighing process was repeated. These actions were repeated with fresh deionized water after every 24 hour soak until the test was terminated or the material completely dissolves.

In Table 2 below, the % mass remaining after four days was calculated by subtracting the % mass lost after four days from 100.

### Injection Force

The force required to eject the graft from a 14 mL syringe (14.4 mm ID) and through a Jamshidi-type needle into a 5 mL or 10 mL cup, or from a 40 mL syringe (24.2 mm ID) into a 50 mL cup, was determined through the following methodology. A specified amount of graft was prepared in a 14 mL or 40 mL syringe. Following mixing, a Jamshidi-type needle was attached via the luer connection of the syringe. The bottom of the needle was positioned 12.7 mm from the bottom of the test cup in an MTS test-frame with a 1 kN load cell. Starting at 5 minutes following combination of the particulate composition component and aqueous solution component of the graft, a force was applied to the syringe/needle system at a rate of 4.4 mm/sec. Force vs. distance data were sampled at a rate of 50 Hz until the specimen was ejected into a 5mL test cup. The resistance force at 15 mm displacement was recorded as the injection force required to eject the specimen.

### Example 1: Groups A-F and Comparator Group J

Vicat set times (both dry and wet), and cement dissolution rate were evaluated for samples of the bone graft formulation with differing liquid to powder ratios (L/P), sorted into Groups A-F and Comparator J. Injection force with approximately 7cc graft in a 14 mL syringe, with an 11 gauge 10 cm needle were tested for the same groups. The particulate make-up of the experimental formulations was maintained for each group and comprised 75% calcium sulfate hemihydrate, 6.7 ± 0.1% monocalcium phosphate monohydrate, 8.3% Beta tricalcium phosphate powder, and 10% Beta tricalcium phosphate granules, (optionally with 0.1% sucrose coated calcium sulfate dihydrate). The L/P was varied between the formulations using different aqueous solutions, which are defined in Table 1 below: ....................

**Table 1 Concentrations of Components in Aqueous Test Solutions**

| **Ingredient** | **AS1** | **AS2** | **AS3₀** | **AS3** | **AS4** |
|---|---|---|---|---|---|
| Crystalline glycolic acid (Wt/V) | 4.562-4.564% | 3.912-3.914% | 3.35-3.38% | 3.07-3.10% | 2.83-2.86% |
| Sodium hydroxide (Wt/V) | 2.390-2.392% | 2.050-2.051% | 1.75-1.77% | 1.61-1.62% | 1.48-1.50% |
| Sodium chloride granules (Wt/V) | - | 0.899-0.901% | - | - | - |
| Water | qs | qs | qs | qs | qs |

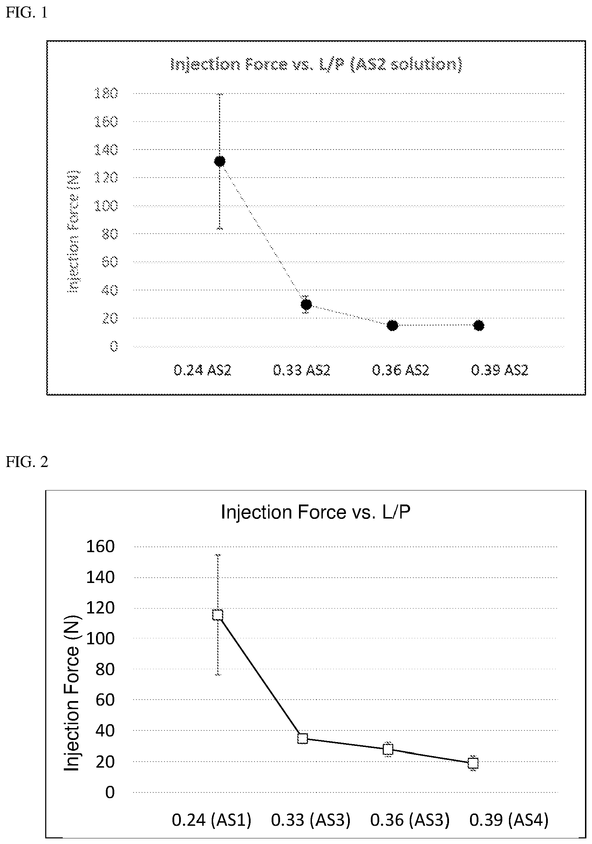

The results of the property testing of the various samples in each of Groups A-F and Comparator Group J were averaged and are provided in Table 2 below with standard deviation. In addition, Figures 1 and 2 graphically illustrate the relationship between injection force of the bone graft substitute formulation and the L/P ratio of the bone graft formulation using AS2, AS3₀, and AS3.

**Table 2**

| **Group No.** | **Aqueous Solution** | **L/P** | **Vicat Set Time (dry) (minutes)** | **Vicat Set Time (wet) (minutes)** | **Dissolution: % mass of cement remaining after 4 days** | **Injection Force (N)** |
|---|---|---|---|---|---|---|
| A | AS1 | 0.36 | 37.3 ± 5.8 (n=6) | not tested | 60.9 ± 2.8 (n=5) | not tested |
| B | AS1 | 0.39 | 41.3 ± 1.5 (n=3) | not tested | 61.2 ± 1.4 (n=5) | not tested |
| C | AS2 | 0.36 | 41.2 ± 9.0 (n=10) | not tested | 60.0 ± 2.5 (n=10) | 15.1 ± 1.4 (n=3) |
| D | AS2 | 0.39 | 42.4 ± 3.9 (n=7) | not tested | 56.9 ± 3.6 (n=10) | 15.5 ± 2.4 (n=3) |
| E | AS3 | 0.36 | 32.6 ± 2.1 (n=9) | 36.9 ± 4.1 (n=9) | 62.0 ± 3.2 (n=15) | 28.0 ± 4.5 (n=9) |
| F | AS4 | 0.39 | 41.0 ± 10.8 (n=12) | 36.7 ± 2.9 (n=9) | 58.3 ± 3.7 (n=15) | 19.1 ± 4.7 (n=9) |
| Comparator J | AS1 | 0.24 | 19.0 ± 2.6 (n=11) | 18.2 ± 1.0 (n=3) | 67.3 ± 5.7 (n=20) | 115.4 ± 39.1 (n=8) |

### Conclusion:

Adjusting the liquid to powder ratio (L/P) to the range of about 0.35 to about 0.40 surprisingly provided bone graft substitute formulations with longer set times while the resulting cement had dissolution rates comparable to the prior formulations of the art with a lower L/P. The bone graft substitute formulations with an L/P ratio of about 0.35 to about 0.40 also demonstrate substantially lower injection forces. The comparable dissolution rates coupled with the longer set times and lower injection forces provides bone substitute graft formulations that can be placed in larger syringes and/or narrower gauge needles for differing bone injuries, and yet provide the same absorption behavior as the prior art cement once set inside the bone needing repair.

### Example 2: Injection Force with Needles of Different Sizes - 14 mL Syringe

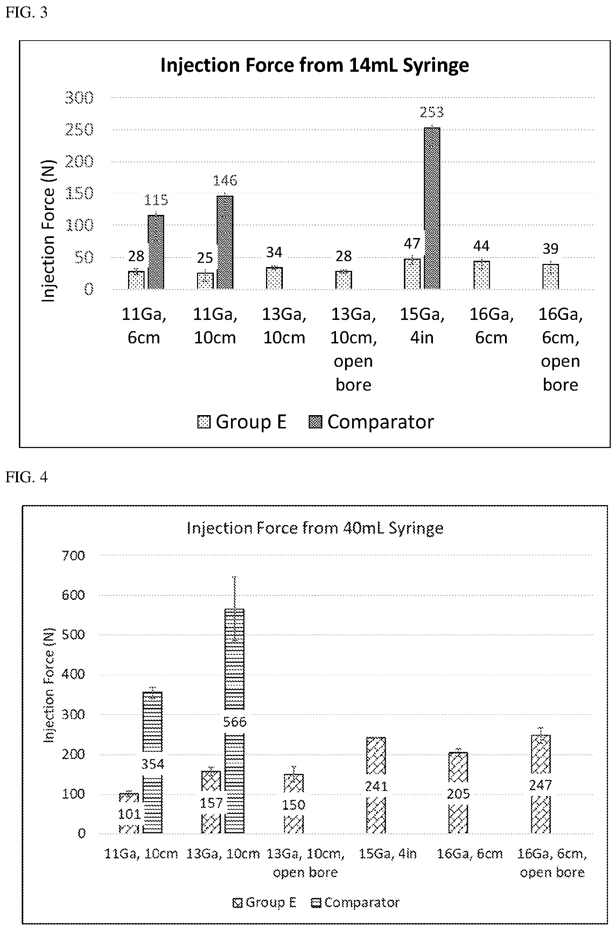

Two grafts prepared with the same amount of powder but differing amounts of solution were prepared in a 14 mL syringe. Unless indicated as an "open-bore" connection, each needle was connected to the syringe via a luer connection. The force associated with injecting approximately 7 cc of Group E graft into a 10 mL cup was measured for the following needle sizes: 11 gage 6 cm (n=8), 11 gage 10 cm (n=3), 13 gage 10 cm (n=3), 13 gage 10 cm - open bore (n=3), 15 gage 4 in (n=3), 16 gage 6 cm (n=2), and 16 gage 6 cm - open bore (n=3). The force associated with injecting approximately 5 cc of Comparator (Group J) graft into a 10 mL cup was tested for the following needle sizes: 11 gage 6 cm (n=9), 11 gage 10 cm (n=3), and 15 gage 4 in (n=3). The results are graphically illustrated in Figure 3.

### Example 3: Injection Force with Needles of Different Sizes Using a 40 mL Syringe

Two grafts prepared with the same amount of powder but differing amounts of solution were prepared in a 40 mL syringe. Unless indicated as "open-bore", each needle was connected to the syringe via a luer connection. The force associated with injecting approximately 25 cc of Group E graft into a 50 mL cup was measured for the following needle sizes: 11 gage 6 cm (n=2), 11 gage 10 cm (n=2), 13 gage 10 cm (n=3), 13 gage 10 cm - open bore (n=3), 15 gage 4 in (n=2), 16 gage 6 cm (n=2), and 16 gage 6 cm - open bore (n=2). The force associated with injecting approximately 20 cc of Comparator (Group J) graft into a 50 mL cup was tested for the following needle sizes: 11 gage 6 cm (n=2), and 11 gage 10 cm (n=2) in Figure 4.

### Example 4: Working Time - Multiple Ejections 14 mL and 40 mL Syringes

A specified amount of graft was prepared in a (14.4 mm ID) or 40 mL (24.2 mm ID) syringe designed to facilitate mixing and delivery of the graft. After preparation of the graft, a needle with an identified gage and cannula length was attached to the syringe. Ejection time-points were identified by the minutes after combination of the solution and powder (i.e., initiation of graft preparation). Ejection was facilitated by compressing the syringe plunger at designated time-points to express 0.4 - 0.8 mL per time-point, at 3, 5, 7 minutes and 1-2 minute intervals thereafter. The following milestones and correspondent time-point were noted: time of first ejection (e.g., 3 minutes); a spindle-driver (to rotate plunger) engaged because hand-compression yielded little to no ejection; too difficult to eject (i.e., little to no ejection including with spindle-driver); and, no remaining visible graft.

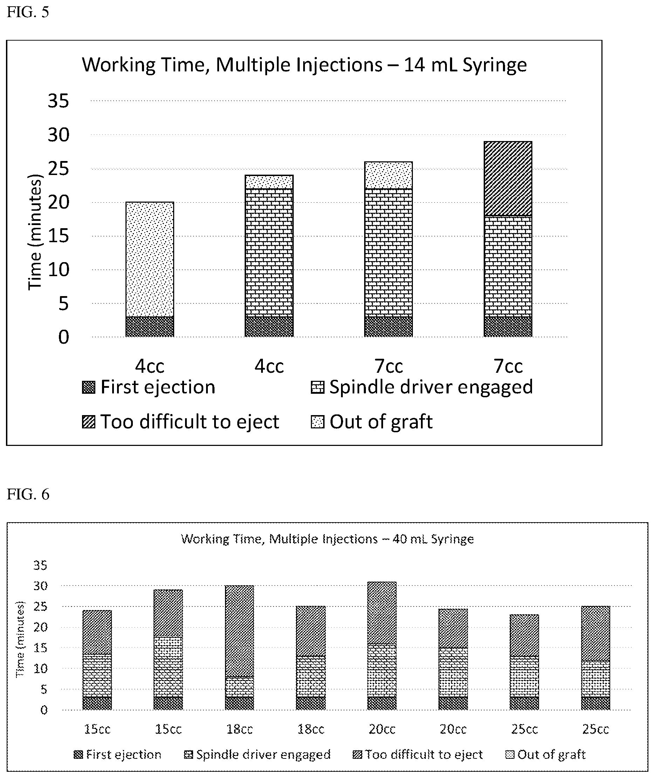

Graft samples of Group E, 4 cc and 7 cc (n=2/volume), were prepared in 14 mL syringes. After preparation, a 13 gage 10 cm needle was attached to the syringe via a luer connector. Results for samples in the 14 mL syringes are graphically presented in Figure 5.

Graft samples of Group E, 15cc, 18cc, 20cc, and 25cc (n=2/volume), were prepared in 40mL syringes. After preparation, a 13 gage 10 cm needle was attached to the syringe via a luer connector. Results for samples in the 14 mL syringes are graphically presented in Figure 6.

### Example 4: Working Time - Single Ejection - 14 mL and 40 mL Syringes

A specified amount of graft was prepared in a 14 mL (14.4 mm ID) or 40 mL (24.2 mm ID) syringe designed to facilitate mixing and delivery of the graft. After preparation of the graft, a needle with an identified gage and cannula length was attached to the syringe via a luer connector; alternatively, a needle with an O-ring around was attached to syringe via an open bore-connector (i.e., luer was removed). Time-points were identified by the minutes after combination of the solution and powder (i.e., initiation of graft preparation). Ejection was facilitated by compressing the syringe plunger at the designated time-point to express all of the visible graft in the syringe. For each time-point, the difficult of expressing all graft via hand compression only (i.e., no use of a spindle driver) was rated on a scale of 1 (very easy) to 10 (very difficult).

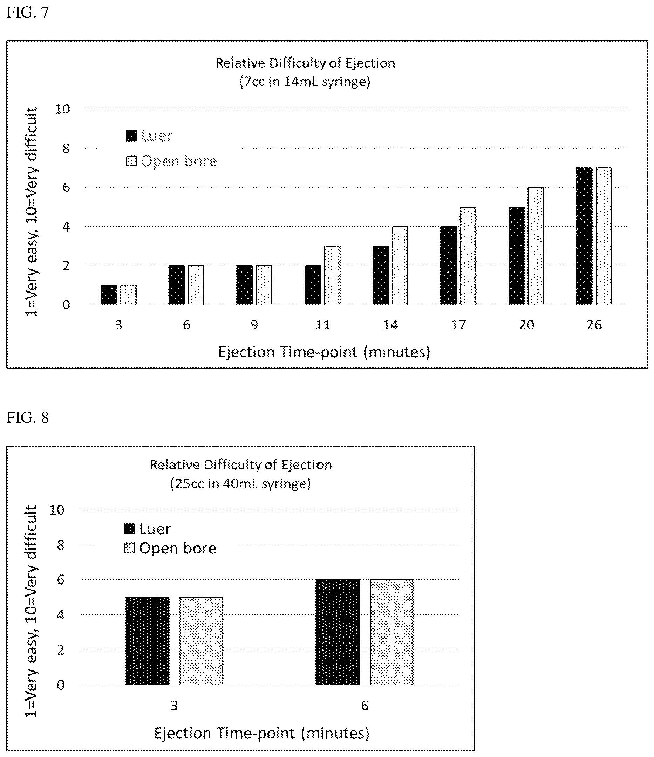

Graft samples of Group E, 7cc, were prepared in 14mL syringes. After preparation, a 13 gage 10 cm needle was attached to the syringe via either a luer connector (n=1/time-point), or an open-bore connector and an O-ring on the syringe (n=1/time-point). Samples were ejected at the following time-points: 3, 6, 9, 11, 14, 17, 20, and 26 minutes. Results for samples ejected from the 14mL syringes are graphically presented in Figure 7.

Graft samples of Group E, 25cc (n=2/volume), were prepared in 40 mL syringes. After preparation, a 13 gage 10cm needle was attached to the syringe via either a luer connector (n=1/time-point), or an open-bore connector and an O-ring on the syringe (n=1/time-point). Samples were ejected at the following time-points: 3 and 6 minutes. Results for samples ejected from the 40 mL syringes are graphically presented in Figure 8.

### Example 5: Treatment of Subchondral Cyst of about 2 mL

A subchondral cyst having a void of about 2 mL may be filled using the bone graft substitute formulation of the present disclosure. At the time of treatment, 6.2 g of particulate composition as described herein is combined with 2.4 g aqueous solution as described herein in a 14 mL mixing syringe. The formulation is then manually delivered from the 14 mL mixing syringe (14.4 mm ID) through a suitable needle, such as a 16 gauge x 6 cm needle; a 16 gauge x 10cm cannula; a 15 gauge x 4 inch needle; a 13 gauge x 10 cm needle; and larger needles with a similar length.

### Example 6: Treatment of Bone Cyst of about 5 mL

A bone cyst having a void of about 5 mL may be filled using the bone graft substitute formulation of the present disclosure. At the time of treatment, 10.9 g of particulate composition as described herein is combined with 4.1 g aqueous solution as described herein in a 14 mL mixing syringe. The formulation is then manually delivered from the 14 mL mixing syringe (14.4 mm ID) through a suitable needle, such as a 16 gauge x 6 cm needle; a 15 gauge x 4 inch needle; a 13 gauge x 10 cm needle; and larger needles with a similar length.

### Example 7: Treatment of Bone Cyst of about 25 mL

A bone cyst having a void of about 25 mL may be filled using the bone graft substitute formulation of the present disclosure. At the time of treatment, 35 g of particulate composition as described herein is combined with 13.2 g aqueous solution as described herein in a 40 mL mixing syringe. The formulation is then manually delivered from the 40 mL mixing syringe (24.2 mm ID) through a suitable needle, such as a 16 gauge x 6 cm needle; a 15 gauge x 4 inch needle; a 13 gauge x 10 cm needle; and larger needles with a similar length.

## Claims

1. A bone graft substitute formulation comprising:
i. a particulate composition component comprising a calcium sulfate hemihydrate powder, monocalcium phosphate monohydrate powder, and a β-tricalcium phosphate powder, wherein the calcium sulfate hemihydrate powder is present in an amount from about 65 weight percent to about 85 weight percent, based on the total weight of the particulate composition component; and
ii. an aqueous component,
wherein the particulate composition component further comprises a plurality of β-tricalcium phosphate granules having a median particle size greater than the median particle size of the β-tricalcium phosphate powder;
wherein the bone graft substitute formulation has a liquid weight to powder weight ratio (L/P) of about 0.35 to about 0.40.

2. The bone graft substitute formulation of claim 1, wherein the particulate composition comprises an accelerant, wherein the accelerant is calcium sulfate dihydrate, potassium sulfate, or sodium sulphate.

3. The bone graft substitute formulation of claim 1 or claim 2, wherein the accelerant is calcium sulfate dihydrate crystals coated with sucrose.

4. The bone graft substitute formulation of claim 2 or 3, wherein the accelerant is present in an amount of up to about 1.0 weight percent, optionally wherein the accelerant is present in an amount from about 0.001 to about 0.5 weight percent, or from about 0.01 to about 0.3 weight percent, based on the total weight of the particulate composition component.

5. The bone graft substitute formulation of any of claims 1-4, wherein the β-tricalcium phosphate granules have a median particle size of about 75 to about 1,000 microns, optionally wherein the β-tricalcium phosphate granules have a median particle size of about 100 to about 400 microns, or about 180 to about 240 microns.

6. The bone graft substitute formulation of any of claims 1-5, wherein the β-tricalcium phosphate granules are present in an amount of up to about 20 weight percent, or up to about 15 weight percent, or up to about 10 weight percent, based on the total weight of the particulate composition component.

7. The bone graft substitute formulation of any of claims 1-6, wherein the aqueous component comprises glycolic acid, optionally further comprising one or more additives selected from sodium chloride, potassium chloride, sodium sulfate, potassium sulfate, EDTA, ammonium sulfate, ammonium acetate, or sodium acetate.

8. The bone graft substitute formulation of any of claims 1-7, further comprising a plasticiser.

9. The bone graft substitute formulation of claim 8, wherein the plasticiser is selected from glycerol and other polyols, vinyl alcohol, stearic acid, hyaluronic acid, cellulose derivatives, and mixtures thereof.

10. The bone graft substitute formulation of claim 8 or claim 9, wherein the plasticiser is an alkyl cellulose, optionally wherein the alkyl cellulose is selected from methylhydroxypropylcellulose, methylcellulose, ethylcellulose, hydroxyethylcellulose, hydroxypropylcellulose, hydroxypropylmethylcellulose, carboxymethylcellulose, cellulose acetate butyrate, and mixtures or salts thereof.

11. The bone graft substitute formulation of any of claims 1-10, further comprising a biologically active agent.

12. The bone graft substitute formulation of claim 11, wherein the biologically active agent is selected from:
(a) peptides, proteins, enzymes, small molecule drugs, dyes, lipids, nucleosides, oligonucleotides, polynucleotides, nucleic acids, cells, viruses, liposomes, microparticles, and micelles;
(b) osteoinductive or osteoconductive materials, antibiotics, chemotherapeutic agents, pesticides, antivirals, anti-inflammatory agents, and analgesics, optionally wherein the biological agent is a pesticide selected from antifungal agents and antiparasitic agents; or
(c) antibodies.

13. A kit comprising the components of the bone graft substitute formulation according to any of claims 1-12, optionally wherein the kit comprises one or more containers enclosing the particulate composition components and one or more separate containers enclosing the aqueous solution, optionally wherein the aqueous solution is sterile.

## Patentansprüche

1. Knochentransplantatersatzformulierung, umfassend:
i. eine partikuläre Zusammensetzungskomponente, die Calciumsulfathemihydrat-Pulver, Monocalciumphosphatmonohydrat-Pulver und β-Tricalciumphosphat-Pulver umfasst, wobei das Calciumsulfathemihydrat-Pulver in einer Menge von etwa 65 Gewichtsprozent bis etwa 85 Gewichtsprozent, bezogen auf das Gesamtgewicht der partikulären Zusammensetzungskomponente, vorliegt; und
ii. eine wässrige Komponente,
wobei die partikuläre Zusammensetzungskomponente ferner eine Vielzahl von β-Tricalciumphosphat-Granulaten mit einer medianen Partikelgröße umfasst, die größer als die mediane Partikelgröße des β-Tricalciumphosphat-Pulvers ist;
wobei die Knochentransplantatersatzformulierung ein Verhältnis von Flüssigkeitsgewicht zu Pulvergewicht (L/P) von etwa 0,35 bis etwa 0,40 aufweist.

2. Knochentransplantatersatzformulierung nach Anspruch 1, wobei die partikuläre Zusammensetzung einen Beschleuniger umfasst, wobei der Beschleuniger Calciumsulfatdihydrat, Kaliumsulfat oder Natriumsulfat ist.

3. Knochentransplantatersatzformulierung nach Anspruch 1 oder Anspruch 2, wobei es sich bei dem Beschleuniger um Calciumsulfatdihydrat-Kristalle handelt, die mit Saccharose beschichtet sind.

4. Knochentransplantatersatzformulierung nach Anspruch 2 oder 3, wobei der Beschleuniger in einer Menge von bis zu etwa 1,0 Gewichtsprozent vorliegt, wobei der Beschleuniger optional in einer Menge von etwa 0,001 bis etwa 0,5 Gewichtsprozent oder von etwa 0,01 bis etwa 0,3 Gewichtsprozent, bezogen auf das Gesamtgewicht der partikulären Zusammensetzungskomponente, vorliegt.

5. Knochentransplantatersatzformulierung nach einem der Ansprüche 1-4, wobei die β-Tricalciumphosphat-Granulate eine mediane Partikelgröße von etwa 75 bis etwa 1.000 Mikrometer aufweisen, wobei die β-Tricalciumphosphat-Granulate optional eine mediane Partikelgröße von etwa 100 bis etwa 400 Mikrometer oder etwa 180 bis etwa 240 Mikrometer aufweisen.

6. Knochentransplantatersatzformulierung nach einem der Ansprüche 1-5, wobei die β-Tricalciumphosphat-Granulate in einer Menge von bis zu etwa 20 Gewichtsprozent oder bis zu etwa 15 Gewichtsprozent oder bis zu etwa 10 Gewichtsprozent, bezogen auf das Gesamtgewicht der partikulären Zusammensetzungskomponente, vorliegen.

7. Knochentransplantatersatzformulierung nach einem der Ansprüche 1-6, wobei die wässrige Komponente Glykolsäure umfasst, optional ferner umfassend ein oder mehrere Additive, ausgewählt aus Natriumchlorid, Kaliumchlorid, Natriumsulfat, Kaliumsulfat, EDTA, Ammoniumsulfat, Ammoniumacetat oder Natriumacetat.

8. Knochentransplantatersatzformulierung nach einem der Ansprüche 1-7, die ferner einen Weichmacher umfasst.

9. Knochentransplantatersatzformulierung nach Anspruch 8, wobei der Weichmacher ausgewählt ist aus Glycerin und anderen Polyolen, Vinylalkohol, Stearinsäure, Hyaluronsäure, Cellulosederivaten und Mischungen davon.

10. Knochentransplantatersatzformulierung nach Anspruch 8 oder Anspruch 9, wobei der Weichmacher eine Alkylcellulose ist, wobei die Alkylcellulose optional ausgewählt ist aus Methylhydroxypropylcellulose, Methylcellulose, Ethylcellulose, Hydroxyethylcellulose, Hydroxypropylcellulose, Hydroxypropylmethylcellulose, Carboxymethylcellulose, Celluloseacetatbutyrat und Mischungen oder Salzen davon.

11. Knochentransplantatersatzformulierung nach einem der Ansprüche 1-10, die ferner ein biologisch aktives Mittel umfasst.

12. Knochentransplantatersatzformulierung nach Anspruch 11, wobei das biologisch aktive Mittel ausgewählt ist aus:
(a) Peptiden, Proteinen, Enzymen, niedermolekularen Arzneimitteln, Farbstoffen, Lipiden, Nukleosiden, Oligonukleotiden, Polynukleotiden, Nukleinsäuren, Zellen, Viren, Liposomen, Mikropartikeln und Mizellen;
(b) osteoinduktiven oder osteokonduktiven Materialien, Antibiotika, Chemotherapeutika, Pestiziden, antiviralen Mitteln, entzündungshemmenden Mitteln und Analgetika, wobei das biologische Mittel optional ein Pestizid ist, das aus antimykotischen Mitteln und antiparasitären Mitteln ausgewählt ist; oder
(c) Antikörpern.

13. Kit, umfassend die Komponenten der Knochentransplantatersatzformulierung nach einem der Ansprüche 1-12, wobei das Kit optional einen oder mehrere Behälter, enthaltend die partikulären Zusammensetzungskomponenten, und einen oder mehrere getrennte Behälter, enthaltend die wässrige Lösung, umfasst, wobei die wässrige Lösung optional steril ist.

## Revendications

1. Formulation de substitut de greffe osseuse comprenant :
i. un composant composition particulaire comprenant une poudre d'hémihydrate de sulfate de calcium, une poudre de monohydrate de phosphate monocalcique et une poudre de phosphate β-tricalcique, dans lequel la poudre d'hémihydrate de sulfate de calcium est présente en une quantité d'environ 65 pour cent en poids à environ 85 pour cent en poids, sur la base du poids total du composant composition particulaire ; et
ii. un composant aqueux,
dans laquelle le composant composition particulaire comprend en outre une pluralité de granules de phosphate β-tricalcique ayant une taille médiane de particule supérieure à la taille médiane de particule de la poudre de phosphate β-tricalcique ;
dans laquelle la formulation de substitut de greffe osseuse a un rapport de poids de liquide sur poids de poudre (L/P) d'environ 0,35 à environ 0,40.

2. Formulation de substitut de greffe osseuse selon la revendication 1, dans laquelle la composition particulaire comprend un accélérateur, dans laquelle l'accélérateur est le sulfate de calcium dihydraté, le sulfate de potassium ou le sulfate de sodium.

3. Formulation de substitut de greffe osseuse selon la revendication 1 ou la revendication 2, dans laquelle l'accélérateur est constitué de cristaux de sulfate de calcium dihydraté revêtus de saccharose.

4. Formulation de substitut de greffe osseuse selon la revendication 2 ou 3, dans laquelle l'accélérateur est présent en une quantité allant jusqu'à environ 1,0 pour cent en poids, facultativement dans laquelle l'accélérateur est présent en une quantité allant d'environ 0,001 à environ 0,5 pour cent en poids, ou d'environ 0,01 à environ 0,3 pour cent en poids, sur la base du poids total du composant composition particulaire.

5. Formulation de substitut de greffe osseuse selon l'une quelconque des revendications 1 à 4, dans laquelle les granules de phosphate β-tricalcique ont une taille moyenne de particule d'environ 75 à environ 1 000 microns, éventuellement dans laquelle les granules de phosphate β-tricalcique ont une taille moyenne de particule d'environ 100 à environ 400 microns, ou d'environ 180 à environ 240 microns.

6. Formulation de substitut de greffe osseuse selon l'une quelconque des revendications 1 à 5, dans laquelle les granules de phosphate β-tricalcique sont présents en une quantité allant jusqu'à environ 20 pour cent en poids, ou jusqu'à environ 15 pour cent en poids, ou jusqu'à environ 10 pour cent en poids, par rapport au poids total du composant composition particulaire.

7. Formulation de substitut de greffe osseuse selon l'une quelconque des revendications 1 à 6, dans laquelle le composant aqueux comprend de l'acide glycolique, éventuellement en outre comprenant un ou plusieurs additifs choisis parmi le chlorure de sodium, le chlorure de potassium, le sulfate de sodium, le sulfate de potassium, l'EDTA, le sulfate d'ammonium, l'acétate d'ammonium, ou l'acétate de sodium.

8. Formulation de substitut de greffe osseuse selon l'une quelconque des revendications 1 à 7, comprenant en outre un plastifiant.

9. Formulation de substitut de greffe osseuse selon la revendication 8, dans laquelle le plastifiant est choisi parmi le glycérol et d'autres polyols, l'alcool vinylique, l'acide stéarique, l'acide hyaluronique, les dérivés de cellulose, et leurs mélanges.

10. Formulation de substitut de greffe osseuse selon la revendication 8 ou la revendication 9, dans laquelle le plastifiant est une alkylcellulose, facultativement dans laquelle l'alkylcellulose est choisie parmi la méthylhydroxypropylcellulose, la méthylcellulose, l'éthylcellulose, l'hydroxyéthylcellulose, l'hydroxypropylcellulose, l'hydroxypropylméthylcellulose, la carboxyméthylcellulose, l'acétate butyrate de cellulose, et leurs mélanges ou sels.

11. Formulation de substitut de greffe osseuse selon l'une quelconque des revendications 1 à 10, comprenant en outre un agent biologiquement actif.

12. Formulation de substitut de greffe osseuse selon la revendication 11, l'agent biologiquement actif étant choisi parmi :
(a) peptides, protéines, enzymes, médicaments à petite molécule, colorants, lipides, nucléosides, oligonucléotides, polynucléotides, acides nucléiques, cellules, virus, liposomes, microparticules et micelles ;
(b) matériaux ostéo-inducteurs ou ostéo-conducteurs, antibiotiques, agents chimiothérapeutiques, pesticides, antiviraux, agents anti-inflammatoires, et analgésiques, éventuellement dans laquelle l'agent biologique est un pesticide choisi parmi les agents antifongiques et les agents antiparasitaires ; ou
(c) anticorps.

13. Kit comprenant les composants de la formulation de substitut de greffe osseuse selon l'une quelconque des revendications 1 à 12, facultativement dans lequel le kit comprend un ou plusieurs récipients renfermant les composants composition particulaire et un ou plusieurs récipients distincts renfermant la solution aqueuse, facultativement dans lequel la solution aqueuse est stérile.
